# EUROPEAN PATENT APPLICATION

(11) **EP 3 900 637 A1**
(43) Date of publication of application: **27.10.2021**
(21) Application number: 20170642.1
(22) Date of filing: 21.04.2020
(51) Int. Cl.: A61B 9/00, A61B 5/00

(54) **A MEDICAL DEVICE FOR APPLYING FORCE TO THE BODY OF A SUBJECT DURING EXAMINATION, TREATMENT OR DIAGNOSIS**

(71) Applicant: Koninklijke Philips N.V., 5656 AG Eindhoven (NL)
(72) Inventor: WRIGHT, Christopher John, 5656 AE Eindhoven (NL); LAWRENSON, Matthew John, 5656 AE Eindhoven (NL)
(74) Representative: Philips Intellectual Property & Standards

(57) **Abstract**

A medical device is for applying a force to the body of a subject by means of a force application surface. A material structure has an input for receiving a manual input force from a user of the medical device, and an output coupled to the force application surface for delivering an output force. The material structure implements a force transfer function which includes a force limiting function, so that a limited known force is applied to the body of the subj ect.

## Description

### FIELD OF THE INVENTION

The invention relates to devices which are operated manually, for example for subject examination or treatment, and which are used to apply a force to the subject.

### BACKGROUND OF THE INVENTION

When medical practitioners test for reflexes or carry out other physical examination tests which involve controlled force application, they are liable to deliver varying force amplitudes, application angles or other parameter variances which are likely to affect the outcome of the test. These variances lead to subjective conclusions which are difficult to compare.

Solutions have been tested which involve instrumented stimulus tools or video analysis to control the different stimulus parameters. Due to difficulty in transferring to clinical reality and cost of implementation, these have had limited clinical impact.

There remains a need for a more reliable and simple way to provide repeatable forces during examination or testing.

### SUMMARY OF THE INVENTION

The invention is defined by the claims.

According to examples in accordance with an aspect of the invention, there is provided a medical device for applying force to the body of a subject during examination, treatment or diagnosis, the device comprising:
a force application surface having a contact area for contacting the body of the subj ect;
a material structure comprising an input for receiving a manual input force from a user of the medical device, and an output coupled to the force application surface for delivering an output force, wherein the material structure has a force transfer function between the input and the output which implements a force limiting function, by limiting the coupling of a manual input force above a threshold to the output.

This device implements a force threshold behavior so that a known force is applied to the body of a subject, even though a variable input force is applied manually by a user (e.g. a medical practitioner). This device enables the medical practitioner to perform a manual procedure, such as subject examination, with a consistently applied force (for example as between different subjects). In this way, the interpretation of the results of different examinations (or treatments or diagnoses) is more consistent.

The material structure for example has a force transfer function between the input and the output which depends on the angle of the manual input force. For example, a low force may result if the force is applied at an incorrect angle.

The material structure may have a force transfer function between the input and the output which depends on the area of application of the force to the body of the subj ect.

The material structure may have a force transfer function between the input and the output which depends on the temporal dynamics of the manual input force. For example it may be desired to apply a force at a certain rate.

Thus, the material structure may be designed to deliver a force in response to the correct area or direction of application and/or temporal characteristics of the manually applied force.

The material structure for example comprises an arrangement of beams. The design of the individual beams and the relationship between the beams determines the properties of the material. The beams for example form repeating compressible units with together form the bulk of the structure.

The device may further comprise one or more sensors for measuring an interaction at the contact area. This may be used to provide feedback to a user.

The one or more sensors for example comprise one or more of:
a stretch sensor;
a force sensor; and
an acoustic sensor.

Another example is a stress sensor which changes color as a function of stress.

The device may comprise a handle and a detachable tool head, wherein material structure is within the handle and the tool head defines the force application surface. The material structure may in this way be used as part of a handle which can then be attached to a set of different tools.

In one set of examples, the device comprises a reflex hammer, comprising a handle, and a hammer head, wherein the contact area comprises a tip of the hammer head. The device thus enables a known and repeatable force to be applied by the reflex hammer.

In another set of examples, the device comprises a physician glove, wherein the contact area comprises finger pads at the ends of the fingers of the glove. The device thus enables a known and repeatable force to be applied by the fingers of the user.

In another set of examples, the device comprises a tapping pad for acoustic lung inspection, wherein the contact area comprises a surface of the tapping pad.

In another set of examples, the device comprises comprise a neurophysiological testing tool, wherein the contact area comprises a tip of the testing tool. The material structure then controls the force that is applied at the tip. The testing tool may comprise a shaft with a dull tip at one end and a sharp tip at an opposite end, wherein the contact area comprises the tips, and a respective material structure is set back from each of the tips. Thus, a pair of tips each have their own respective material structure.

In another set of examples, the device comprises a tool having a gripping region where the tool is to be gripped by the user, wherein the material structure is provided between the gripping region and a remainder of the tool. Thus, the gripping force applied to the tool is regulated by the material structure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a first example of a medical device in accordance with the invention in the form of a reflex hammer;
Figure 2 shows a second example of a medical device in accordance with the invention in the form of a physician glove;
Figure 3 shows a third example of a medical device in accordance with the invention in the form of a neurophysiological testing tool;
Figure 4 shows a fourth example of a medical device in accordance with the invention in the form a generic tool having a grip;
Figure 5 shows an example structure for a simple linear force limiting device;
Figure 6 shows a design and manufacturing system for the material structure; and
Figure 7 shows a design and manufacture method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a medical device for applying a force to the body of a subject by means of a force application surface. A material structure has an input for receiving a manual input force from a user of the medical device, and an output coupled to the force application surface for delivering an output force. The material structure implements a force transfer function which includes a force limiting function, so that a limited known force is applied to the body of the subject.

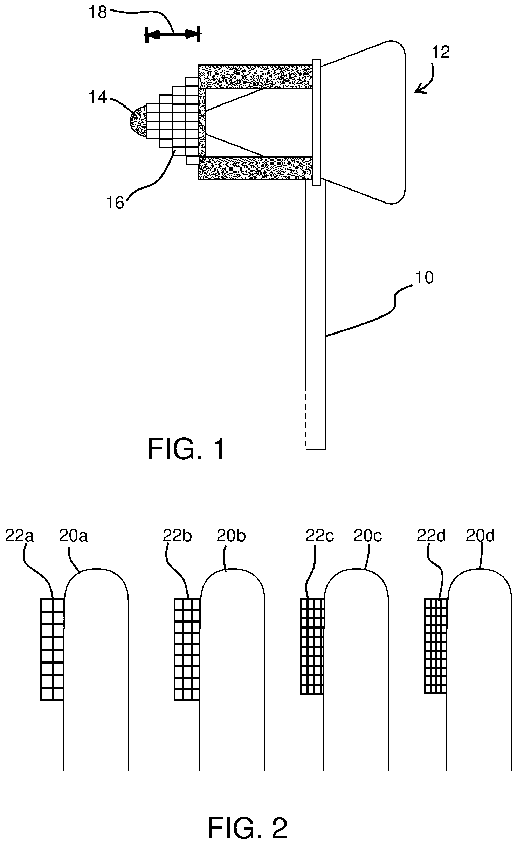

Figure 1 shows a first example of a medical device in accordance with the invention for applying force to the body of a subject during examination, treatment or diagnosis. This example is a reflex hammer with a handle 10 and a hammer head 12. The hammer head 12 has a contact area for contacting the body of the subject (e.g. the knee) at a tip 14 of the hammer head. The contact area is formed by a force application surface, and is the area intended for contact with the human body or with another object.

A material structure 16 is provided between the tip 14 and a rear part of the hammer head. The material structure 16 has an input (the right side in Figure 1) for receiving a manual input force from a user of the hammer, and an output (the left side in Figure 1) coupled to the force application surface, i.e. the tip 14.

The material structure 16 has a force transfer function between the input and the output which implements a force limiting function, by limiting the coupling of a manual input force above a threshold to the output. In particular, the material structure has a range 18 of deformation which corrects the margin of error of the medical practitioner.

The deformation is elastic, so that the device may be re-used repeatedly.

The invention may be applied as a module which is added to an existing reflex hammer surface. Optionally, the material structure 16 has a deformation threshold when shear stress is introduced (which arises when hammer contact is made at an incorrect angle). This threshold may be designed to be much lower so as to induce quick collapse when the incorrect technique is used. This will induce an obvious material collapse without delivering significant force to the subject. The correct technique of applying a perpendicular force delivery to the patient is thereby ensured.

The way the forces are processed by the material structure are part of the design process of the material structure, as described below. In particular, the expected input forces form part of the initial constraints of the design process, and these may include the expected range of force application, and the expected application angle variability (which GPs typically apply when performing reflex tests). These initial conditions may be determined by laboratory condition testing, or through methods of extracting the information from natural videos of GPs performing reflex tests.

The material structure for example comprises a modular arrangement of units, such as beams. The material structure for example does not deform until a pre-defined force threshold is reached, whereupon it deforms in a number of steps as each unit reaches the buckling threshold. The applied force will remain approximately constant even as the material deforms. Using smaller length scales of each unit, e.g. 20 layers of internal repeating units rather than 4 (as schematically shown in Figure 1), will increase the smoothness of the applied force at the chosen maximum. A smooth force application can thereby be achieved based on the force variability defined as part of the initial design conditions.

The tip, which defines the contact area, may be formed of a separate, simple material which fixed to the material structure (as shown in Figure 1) or it may be a surface of the material structure itself. The area of contact or the shape of the contact does not therefore need to be constrained by the design of the material structure.

The back of the material structure is for example rigidly fixed to the reflex hammer head so that force is directly transferred from the handle 10 (the source of the impulse) to the material structure 16. The material structure may be designed to be fixed to a range of different hammer designs for example using an adjustable strap.

Figure 2 shows a second example of a medical device in accordance with the invention in the form of a physician glove. Four fingers 20a to 20d are shown, each with a contact area defined by finger pads 22a to 22d at the ends of the fingers of the glove.

This enables the physician to feel when a preset (threshold) force has been exceeded. The force thresholds, or other characteristics of the force transfer function, may be different between the different fingers of the glove, allowing the doctor to test a range of force sensitivity thresholds. This is shown schematically in Figure 2. Each finger pad for example has a rigid backing to ensure correct functioning of the unit.

The material structure may be designed such that the deformation process changes the topology of the surface contacting the user's skin, such as forming a 'bump', or producing a tap against the surface of the finger pad when the force threshold is reached or the maximum deformation is reached. To achieve this, the response of the material can be designed taking account of the characteristics of the force applied to the rear surface. This may be used to enhance feel-ability of the desired force thresholds being met.

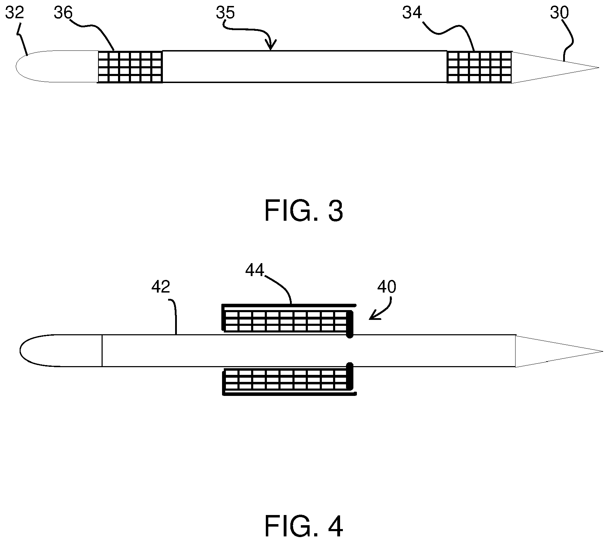

Figure 3 shows a third example of a medical device in accordance with the invention in the form of a neurophysiological testing tool having a sharp end 30 and a blunt/dull end 32. The material structure comprises a first part 34 between the sharp end 30 and the area 35 of the tool which is gripped, and a second part 36 between the blunt end 32 and the area 35 of the tool which is gripped.

The material structures may be permanently fixed in place, or the design may be modular, such that a module with a desired force transfer function may be attached to the grip area 35 to allow testing of different patient sensation force thresholds or other force parameter variations.

Figure 4 shows a fourth example of a medical device in accordance with the invention in the form a generic tool having the material structure 40 formed as a module which may be applied around the outside of an existing unmodified tool 42 (shown by way of example as a neurophysiological testing tool). Pressure applied to a gripping region 44 and then through the material, over the desired threshold, will cause deformation of the grip. This grip structure can be applied to any existing sharp/dull testing tool or also other devices where the applied pressure should be limited.

The example in Figure 4 uses compressive buckling, but examples may use shear buckling. In this case, the material structure may be attached via friction along the whole length of the tool, and a shear force between the gripped area and the tool shaft will cause buckling of unit cell layers. A simple neurological testing tool may be used for several force threshold sensitivity tests by applying different force threshold grips.

The material structure is an example of a so-called mechanical metamaterial. Mechanical metamaterials can be created which have tailorable and exotic mechanical properties, using algorithmic design processes. These include the ability to elastically deform a structure under various load conditions such that the force is carried in pre-defined paths, and the ability to create materials which behave very differently depending on the direction of an applied force (mechanically non-reciprocal).

Existing metamaterials have been characterized where any force above a chosen threshold causes deformation of the material rather than further force transferal. This is the main material property used in the examples above.

For example, the article "Mechanical Performance of Multidirectional Buckling-Based Negative Stiffness Metamaterials: An Analytical and Numerical Study" Materials (Basel) 2018 Jul; 11(7): 1078 (doi: 10.3390/ma11071078) discloses an arrangement by which a force threshold deformation behavior is achieved, using internal curved beams with buckling points. The buckling force threshold of the beams is controlled by the curvature, length, stiffness and thickness properties of the beams. Multiple buckling units can then be arranged in series to achieve a relatively consistent force output during an impact which will not increase beyond an initial threshold until the structure bottoms out.

These effects are achieved through the control of the internal structure of the material. Individually controlling properties such as strut angle, thickness, length and stiffness throughout the material allows the creation of materials with exotic mechanical properties.

Metamaterials with certain target properties may be designed using existing design processes such as machine learning, which can be used to explore large parametric design spaces and even design metamaterial structures from scratch. In addition, trial and error parametric exploration methods may be used to optimize the design within a given set of constraints, such as the design of an initial metamaterial repeating unit with some adjustable parameters.

Units may be formed from the struts, such as cubes. These units are in a repeating manner. For a given repeating unit, such as a cubes with particular bending dynamics, an FEM model (or other modelling technique) may be used to simulate a variety of arrangements of adjustable parameters of the units (strut parameters, unit size etc.), and the parameters of a bulk module (number and physical arrangement of the units) relative to the physical constraints for the particular application.

Force application scenarios may be simulated in this way and analyzed relative to desirable parameters. The solution space may be efficiently explored using Monte-Carlo or other parametric optimization processes.

A range of possible force transfer functions are for example demonstrated in the article "Mechanical Performance of Multidirectional Buckling-Based Negative Stiffness Metamaterials: An Analytical and Numerical Study", referenced above. Once the maximum number of layers have collapsed, the ability of the material to control force application stops. However, it is shown that a fairly flat force response can be achieved with a large number of layers. The actual achievable flatness of the force transfer function will be determined by the capabilities of the manufacturing process (a totally flat response is only achievable in theory with infinite layers). The range of force buffering performed by the material structure is thus selected to fall within the range typically applied by the a user of the device (e.g. a GP).

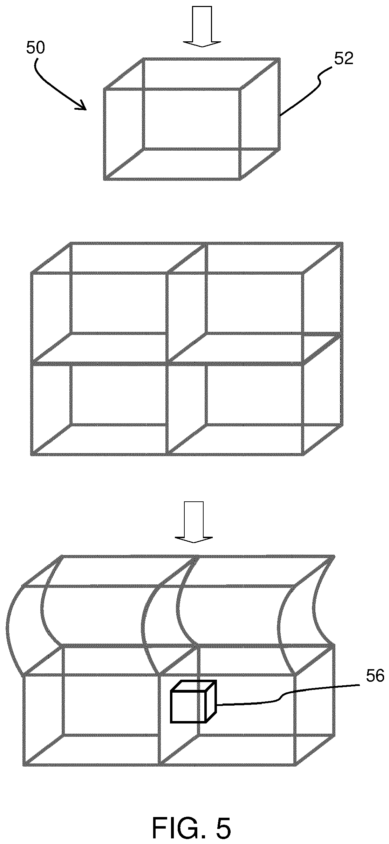

Figure 5 shows an example structure for a simple linear force limiting structure. The basic design building block 50 shown at the top is a cube (or cuboid) formed from struts 52. This unit structure is repeated to form a layered larger material structure 54, shown in the second image down. When a force is applied, the cubes deform layer by layer once a force threshold is reached as shown by the bottom image.

The cubes are for example 0.1 to 10mm in height. The larger end of this scale may be more cost effective to produce but will result in less of a flat force response curve. The dimensions of the struts will depend on the material being used and its properties. It will also vary as a function of the size of the cubes. To allow sufficient ability for the height of each cube to collapse, the struts must preferably be less than 25% of the height of the cube in width (the width of two such struts will take up 50% of the total cube height, leaving 50% collapsible space)

Figure 5 also shows that a sensor 56 may be integrated into the structure.

For example, a strain sensor may be be affixed to any strut or multiple struts in a region of the structure which is highly responsive to the pressure condition at the contact area. This may for example be over the midsection of a strut where bending will occur.

For practicality, the material structure may be coated in an outer membrane to prevent dust ingress etc. If the mechanics of this membrane is also included in the material design process, it could also form a suitable and accessible substrate to which a sensor may be attached.

New fabrication techniques including 3D printing are enabling fast and cheap production of materials with complex internal structures, including mechanical metamaterials. One key development in this space is holographic printing, which allows the printing of an entire 3D structure simultaneously, reducing the print time dramatically.

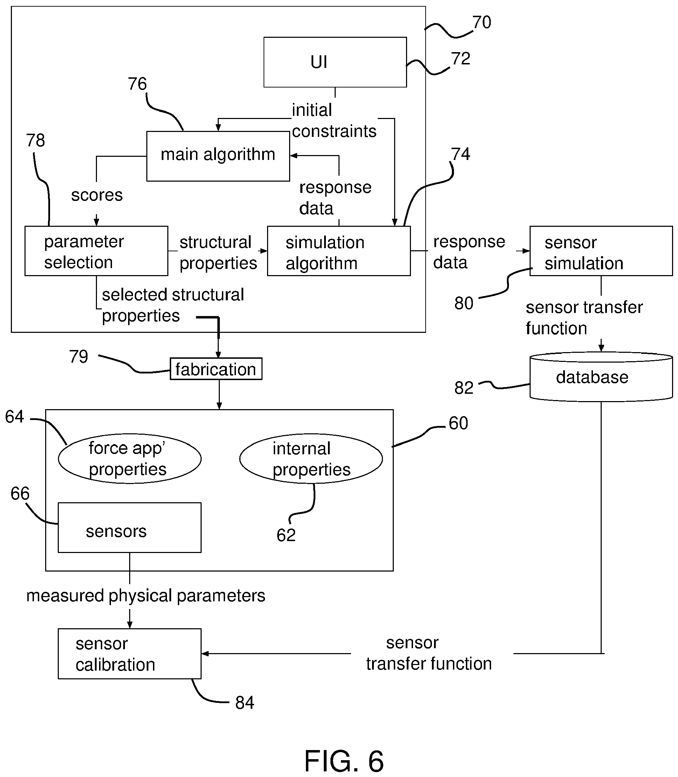

A design and manufacturing system for the material structure will now be described with reference to Figure 6.

The material structure 60 is for mechanical stimulus delivery, and it has tailored internal structural properties 62 such that it transforms the properties of an uncontrolled input force in order to deliver a mechanical stimulus with desirable and consistent force application properties 64.

As discussed in some of the examples above, the force application properties may include:
(i) a force transfer function which limits force amplitudes above a certain threshold.
(ii) dependency of the delivered force on an angle of force application. For example, the structure may deform at a low force threshold if an incorrect application angle is used, preventing a successful reflex response from occurring.
(iii) dependency of the delivered force on an area of force application. In most cases, this area will be defined by the size of the contact area of the material structure . The pressure function of the force application is preferably controlled, which is critical to producing a controlled stretch effect in the patient tissues. There may also be examples where the contact area is larger than the area of force application, where the pressure applied in different ways on the material structure causes a smaller area of force application than the contact area. For example, inward collapse of cubes (or other shapes) around the axis of force application may change the area, and cause one region of the material structure to become stronger than the collapsed regions around it.
(iv) dependency of the delivered force on the temporal dynamics of force application. This may be used to produce a controlled effect on the biological tissue where the force is being applied. The time that the tissue is at maximum stretch during a tap with a reflex hammer for example may be an important factor for effecting the reflex response.
(v) other constraints which may be desirable for controlled stimulus delivery.

The material structure has a complex internal structure typically comprising repeating or non-repeating units as explained above. In a simplest embodiment, repeating units only are employed. These units have defined structural properties and structural arrangements forming the internal structural properties 62 which result in the emergent force application properties 64 of the material structure 60. These properties may include for example, properties of a repeating cube unit which is composed of beams connecting the corners of the cube shape, where the individual beams have properties:
beam length;
beam curvature;
beam thickness;
beam material / stiffness.

The material structure may include sensors 66 which are placed at remote locations relative to the contact area within or on the material structure. These sensors 66 measure physical parameters (the "measured physical parameters") which are related to the force at the contact area by the material structure.

The sensors enable feedback to be provided to the user.

These sensors may be digital sensors, such as stretch sensors, pressure sensors and acoustic sensors or other digital sensors. They may be analog sensors, for example a material which changes color in response to stress. Visual feedback could thereby be given to the user through structural deformations.

A design algorithm 70 is used to design the internal structural properties such that the force application properties are achieved by the material structure. The design algorithm may include a user interface 72 such that a user can input constraints which limit the design space. These inputs may be input by a human designer, and define initial constraints of the design process, such as the dimensions of the material structure external shape and dimensions, and the acceptable force application properties or acceptable ranges of forces.

The desired force application properties may include the desired behavior under specified conditions which relate to the implementation scenario, such as the force reaches but does not exceed a specified response force value under defined conditions.

An example of a defined condition is that the material structure should be compressed between an unconstrained 100g mass travelling at 1m/s and a compressible surface (with the properties of human tissue), where the motion is orthogonal. This is an exemplary scenario which may be analogous to the impact of a reflex hammer. If the motion is non-orthogonal, e.g. occurring at greater than 10 degrees from perpendicular, a new maximum force threshold and therefore material behavior may be defined when the input force is applied beyond this angle.

There are also sensor related constraints, such as the number of sensors, the measured physical parameters, and the location of the sensors.

Other limiting conditions may be defined for practicality such as total material structure weight, limited material options or other properties.

A simulation algorithm 74 simulates material structures with possible combinations of internal structural properties, and generates the response of the resultant material to physical interactions (the "response data"). The nature of the simulated physical forces is defined by the initial constraints.

A main algorithm 76 assesses the quality of the simulated material structure performance relative to the desired force application properties defined in the initial constraints. Each simulated material structure (defined by a combination of internal structural properties) will thereby be given a performance score which may for example be a number between 0 and 1.

When the scoring of different possible designs is complete, a parameter selection algorithm 78 selects the combination of internal structural properties to be simulated and fabricated. The fabrication takes place in step 79.

A sensor simulation algorithm 80 optionally is included to calculate the expected transfer functions which define the correspondence between simulated measured physical parameters at the location of the sensors, and the contact force at the contact area. The sensor transfer functions are stored in a sensor transfer function database 82.

A sensor calibration algorithm 84 determines the actual physical parameter at the contact area (e.g. force applied in the main embodiment) from the measured physical parameters collected by the sensors. Thus, the sensors are employed during use of the fabricated structure, and the simulation processes enable the measured sensor signals to be converted to actual applied force characteristics.

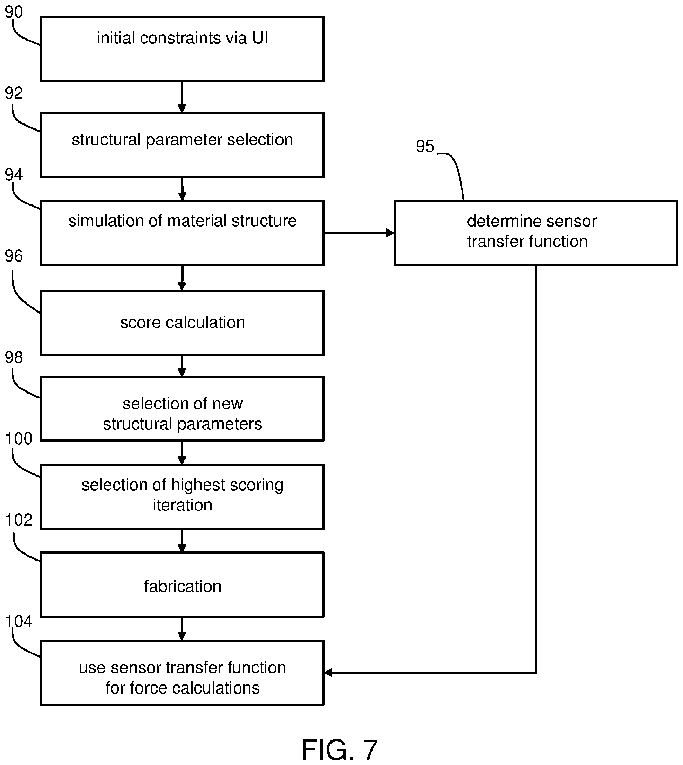

Figure 7 shows the design method.

In step 90, a user inputs the initial constraints via the user interface.

The design algorithm creates an appropriate design for the material structure in terms of its internal structural properties whereby in step 92 the parameter selection algorithm selects a set of initial internal structural properties randomly.

In step 94, the simulation algorithm simulates a material structure with the chosen internal structural properties by using standard FEM modelling software. A model of the material structure is created where the unit is exposed to simulated forces which are derived from the initial constraints and which reflect the implementation scenario of the material structure.

Response data is generated from the software by extracting the modelled forces for areas of interest within the model. In the main embodiment, these reporter locations and attributes will reflect the locations of desired force application properties as they are defined in the initial constraints. For example, these may be:
a point on the contact area measuring force in 3 dimensions; or
points where sensors may be placed (user defined).

In step 96, the main algorithm calculates a material structure score by comparing the response data to the desired force application properties defined in the initial constraints. A strength of match may be calculated as a function of the number of different target parameters.

In step 98, the parameter selection algorithm then selects a new set of internal structural properties for simulation.

The selection of these will be based on the material structure scores for all simulations performed already. Calculation of the new set will be performed by a parametric space exploration algorithm to enable efficient finding of the optimal design for the least computational effort. An example is the Monte-Carlo method. The method may repeat from step 94 until the incremental change in material structure score has reduced below a preset level.

The combination of internal structural properties with the highest material structure score is then selected for fabrication in step 100.

The sensor simulation algorithm determines the sensor transfer function in step 95, for example by determining a multiplication factor which must be applied to measurable forces in the location of the sensor to match the forces of interest in the contact area. More complex transfer functions may be determined mathematically also, according to need.

The sensor transfer function is stored in the sensor function database. The design is then created in physical space by a manufacturing method such as 3D printing in step 102.

The sensor transfer function is applied during use/testing of the manufactured device in step 104. During this use, the sensors collect measured physical parameters and the sensor calibration algorithm calibrates the sensor using the sensor transfer function which corresponds to the material structure embodiment.

The initial constraints are described above as input via a user interface. There may additionally be automated initial constraint extraction from GP interactions. For example, the system may use video or other data which is collected from GP surgeries, where the physical interactions of the doctor and patient are measured to determine force application parameters and variability.

This data may be determined from appropriate force sensors located on GP tools. These calculations may be performed generically for all physicians, or personally on a per-doctor basis. These measurements may then be input as initial constraints to the design algorithm.

The initial constraints may not contain location data for the sensors in some embodiments. These may be calculated algorithmically, where the response data includes many data points across the material structure surface. The data point with the greatest correlation values relative to the forces applied at the contact area may be chosen as the location for sensors thus providing automated sensor position detection.

In summary, the invention provides a device which uses a mechanical material structure which ensures a stimulus application does not exceed preset limits. Excess force may be dissipated in a way which does not affect the critical stimulus parameters (e.g. elastically dissipated or directed away from the force application area).

In addition, the key force parameters of the stimulus may be measured by sensing structures placed within the material structure design. The design of the material structure may be constructed such that specific force application parameters may be monitored from locations which are remote to the area of force application.

The material structure has a volume for mechanical stimulus delivery, which has tailored internal structural properties such that it transforms the properties of an uncontrolled input force in order to deliver a mechanical stimulus with desirable and consistent properties. The design algorithm ensures that the bulk properties of the material structure achieve a set of desired properties for application scenarios, such as for a reflex hammer, gloves, neurological testing tools or other GP-patient interaction scenarios.

The invention may be used for any application where it is desirable to limit forces to a pre-defined level. This is important in applications where an applied force undergoes significant variation, for example through human error. The application of the system to diagnostic tools allows a physician to have certainty of the exact magnitudes of the applied forces despite variation in the forces applied by the physician. More objective conclusions about the patient responses to the applied stimulus can thereby be drawn.

In addition to eliminating force input variability (within a reasonable range of applied forces), stimulus angle variability can also be eliminated as explained above due to the differential behavior of the material under different angles of force application. The placement of distant sensors provides a greater area for the sensors to be placed and prevents interference of the sensing structures with the applied forces.

The invention enables existing tools to be used by providing modules which may be added to those tools. Doctors may therefore use familiar form factors with added functionality.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A medical device for applying force to the body of a subject during examination, treatment or diagnosis, the device comprising:
a force application surface (14) having a contact area for contacting the body of the subject;
a material structure (16) comprising an input for receiving a manual input force from a user of the medical device, and an output coupled to the force application surface (14) for delivering an output force, wherein the material structure has a force transfer function between the input and the output which implements a force limiting function, by limiting the coupling of a manual input force above a threshold to the output.

2. A medical device according to claim 1, wherein the material structure (16) has a force transfer function between the input and the output which depends on the angle of the manual input force.

3. A medical device according to claim 1 or 2, wherein the material structure (16) has a force transfer function between the input and the output which depends on the area of application of the force to the body of the subject.

4. A medical device according to any one of claims 1 to 3, wherein the material structure (16) has a force transfer function between the input and the output which depends on the temporal dynamics of the manual input force.

5. A medical device according to any one of claims 1 to 4, wherein the material structure (16) comprises an arrangement of beams (52).

6. A medical device according to any one of claims 1 to 5, further comprising one or more sensors (56) for measuring an interaction at the contact area.

7. A medical device according to claim 6, wherein the one or more sensors comprise one or more of:
a stretch sensor;
a force sensor; and
an acoustic sensor.

8. A medical device according to claim 6 or 7, wherein the one or more sensors comprise a stress sensor which changes color as a function of stress.

9. A medical device according to any one of claims 1 to 8, comprising a handle and a detachable tool head, wherein material structure is within the handle and the tool head defines the force application surface.

10. A medical device according to any one of claims 1 to 9, comprising a reflex hammer, comprising a handle (10) and a hammer head (12), wherein the contact area comprises a tip (14) of the hammer head.

11. A medical device according to any one of claims 1 to 9, comprising a physician glove, wherein the contact area comprises finger pads (22a-22d) at the ends of the fingers (20a-20d) of the glove.

12. A medical device according to any one of claims 1 to 9, comprising a tapping pad for acoustic lung inspection, wherein the contact area comprises a surface of the tapping pad.

13. A medical device according to any one of claims 1 to 9, comprising a neurophysiological testing tool, wherein the contact area comprises a tip (30,32) of the testing tool.

14. A medical device according to claim 13, comprising a shaft with a blunt tip (32) at one end and a sharp tip (30) at an opposite end, wherein the contact area comprises the tips, and a respective material structure (36,34) is set back from each of the tips.

15. A medical device according to any one of claims 1 to 9, comprising a tool (42) having a gripping region (44) where the tool is to be gripped by the user, wherein the material structure (40) is provided between the gripping region and a remainder of the tool.
